(19) Europäisches Patentamt
European Patent Office
Office européen des brevets

(11) Publication number: **0 469 017 B1**

(12) **EUROPEAN PATENT SPECIFICATION**

(45) Date of publication of patent specification: 15.06.94

(51) Int. Cl.5: **C12N 15/81**, C12N 15/62, C12N 15/12, C07H 21/00, C12N 1/18, C12P 21/02, A61K 37/00

(21) Application number: **90906181.4**

(22) Date of filing: **17.04.90**

(86) International application number:
**PCT/EP90/00617**

(87) International publication number:
**WO 90/12879 (01.11.90 90/25)**

(54) **EXPRESSION OF HUMAN APOLIPOPROTEINS AI-MILANO IN YEAST.**

(30) Priority: **20.04.89 IT 2022689**

(43) Date of publication of application:
**05.02.92 Bulletin 92/06**

(45) Publication of the grant of the patent:
**15.06.94 Bulletin 94/24**

(84) Designated Contracting States:
**AT BE CH DE DK ES FR GB IT LI LU NL SE**

(56) References cited:
**EP-A- 0 293 357**
**WO-A-87/02062**
**WO-A-88/03166**
**WO-A-89/02463**
**US-A- 4 546 082**

**Chemical Abstracts, vol. 109, no. 15, 10 Oct. 1988, (Columbus, Ohio, US), p. 199, abstract 123809x**

(73) Proprietor: **SIRTORI, Cesare**
**Corso Monforte, 36**
**I-20100 Milano(IT)**

Proprietor: **FRANCESCHINI, Guido**
**Via Pancaldo, 12**
**I-20100 Milano(IT)**

(72) Inventor: **SIDOLI, Alessandro**
**Via Friuli 75**
**I-20100 Milano(IT)**
Inventor: **BARALLE, Francisco**
**Residenza Acacie 112**
**Milano 3(IT)**
Inventor: **Sirtori,Cesare**
**Corso Monforte,36**
**I-20100 Milano(IT)**
Inventor: **Franceschini,Guido**
**Via Pancaldo,12**
**I-20100 Milano(IT)**

Gene, vol. 54, 1987, Elsevier Science Publishers B.V., (Amsterdam, NL), R.S. Sidhu et al., pp. 175-184

(74) Representative: **Bianchetti, Giuseppe**
**Studio Consulenza Brevettuale,**
**Via Rossini, 8**
**I-20122 Milan (IT)**

**Description**

The present invention refers to the expression of apolipoprotein AI or apolipoprotein AI-Milano (Apo-AI or Apo-AI-M) in yeast strains and to pharmaceutical preparations containing as active principle said apolipoproteins for the treatment of atherosclerosis and cardiovascular diseases.

The clear correlation between elevated levels of serum cholesterol and the development of coronary heart disease (CHD) has been repeatedly confirmed, based on epidemiological and longitudinal studies. The definition, however, of complex mechanisms of cholesterol transport in plasma, has allowed to recognize a selective function of circulating lipoproteins in determining the risk of CHD.

There are, in fact, four major circulating lipoproteins: chylomicrons (CM), very low density (VLDL), low density (LDL) and high density (HDL) lipoproteins. While CM-constitute a short-lived product of intestinal fat absorption, VLDL and, particularly, LDL are responsible for the cholesterol transport into tissues, among these, also into the arterial walls. In contrast, HDL are directly involved in the removal of cholesterol from peripheral tissues, carrying it back either to the liver or to other lipoproteins, by a mechanism known as "reverse cholesterol transport" (RCT).

The "protective" role of HDL has been confirmed in a number of studies (Miller et al., Lancet, i: 965-968, 1977; Whayne et al., Atherosclerosis 39: 411-419, 1981). In these, the elevated levels of LDS, less so of VLDL, seem to be clearly associated with an increased cardiovascular risk.

Recent interest in the study of the protective mechanism/s of HDL has been focussed into apolipoprotein AI (Apo AI), the major component of HDL. Plasma levels of Apo AI also bear, in fact, a negative correlation with the risk of CHD and with the presence of coronary lesions (Maciejko et al., N.Eng. J. Med., 309: 385-389, 1983; Sedlis et al., Circulation, 73: 978-984, 1986).

In vitro studies, indicate that complexes of Apo AI and lecithin can promote the efflux of free cholesterol from cultured arterial smooth muscle cells (Stein et al., Biochem. Biophys. Acta, 380: 106-118, 1975). By this mechanism HDL can also reduce the proliferation of these cells (Yoshida et al., Exp. Mol. Pathol., 41: 258-266, 1984).

More recently, the infusion of Apo AI or of HDL in experimental animals has been shown to exert significant biochemical changes, as well as to reduce the extent and severity of atherosclerotic lesions. After an initial report by Maciejko and Mao (Arteriosclerosis, 2: 407a, 1982), Badimon et al., in two successive studies (Cardiovascular Disease, 1983, Abs. 81; Arteriosclerosis, 7: 522a, 1987), clearly showed that, by infusing either Apo AI or HDL (d = 1.063-1.325 g/ml), one could significantly reduce the extent of atherosclerotic lesions in cholesterol-fed rabbits (-45%), while reducing the cholesterol ester content in the lesions by 58.4%. It could also be shown (Esper et al., Arteriosclerosis, 7: 523a, 1987) that infusions of HDL can markedly change the plasma lipoprotein composition of Watanabe rabbits with inherited hypercholesterolemia, which develop early arterial lesions. In these, HDL infusions can more than double the ratio between the protective HDL and the atherogenic LDL.

The potential of HDL to improve arterial disease in animal models has been further stimulated by the observation that Apo AI can exert a significant fibrinolytic activity (Saku et al., Thromb. Res., 39: 1-8, 1985). Ronneberger (Xth Int. Congr. Pharmacol., Sidney, 1987, p 990) clearly demonstrated that extractive Apo AI can significantly increase fibrinolysis in beagle dogs and in Cynomolgous monkeys. A similar activity can be noted in vitro on human plasma. This Author could confirm a reduction of lipid deposition and arterial plaque formation in Apo AI treated animals.

Plasma Apo AI is a single polypeptide chain of 243 amino acids, whose primary sequence is known (Brewer et al., Biochem. Biophys. Res. Commun., 80: 623-630, 1978). Apo AI is synthesized as a 267 amino acid precursor in the cell. This pre-pro-apoliprotein first loses the 18 N-terminal residues intracellularly; the loss of 6 further amino acids occurs in the plasma and/or lymph by the activity of specific proteases.

The major structural requirement of the Apo AI molecule is believed to be the presence of repeat units of 11 or 22 aminoacids, presumed to exist in amphipathic helical conformation (Segrest et al., FEBS Lett., 38: 247-253, 1974). This structure allows for the main biological activities of Apo AI, i.e. lipid binding and lecithin cholesterol acyl transferase (LCAT) activation. The other functions of HDL , i.e. interaction with cells and removal of cholesterol from these, are not associated with clear features of the apolipoprotein.

The apolipoprotein AI-Milano (Apo AI-M) is the first described molecular variant of human Apo AI (Franceschini et al., J. Clin. Invest., 66: 892-900, 1980). It is characterized by the substitution of Arg 173 with Cys (Weisgraber et al., J. Biol. Chem., 258: 2508-2513, 1983). The mutant apoprotein is transmitted as an autosomal dominant trait and 8 generations of carriers have been identified (Gualandri et al., Am. J. Hum. Genet., 37: 1083-1097, 1984).

The carrier status of Apo AI-M is characterized by a remarkable reduction of HDL-cholesterol. In spite of this, the affected subjects do not apparently show any increased risk of arterial disease; indeed, by examination of the genealogic tree it appears that these subjects may be "protected" from atherosclerosis.

The mechanism of the possible protective effect of Apo AI-M in the carriers seems to be linked to a modification in the structure of the mutant apolipoprotein, with the loss of one α-helix and an increased esposure of hydrophobic residues (Franceschini et al., J. Biol. Chem., 260: 1632-1635, 1985). The loss of the tight structure of the multiple α-helices leads to an increased flexibility of the molecule, which associates more readily with lipids, compared to normal AI. Moreover, apolipoprotein/lipid complexes are more susceptible to denaturation, thus suggesting that lipid delivery is also improved in the case of the mutant.

Another, very specific feature of the Apo AI-M, is its capacity to form dimers with itself and complexes with Apo AII, in both cases because of the presence of the Cys residue. The presence of dimers and complexes in the circulation is probably responsible for the prolonged elimination half-life of these in the carriers, recently described in clinical studies (Gregg et al., NATO ARW on Human Apolipoprotein Mutants: From Gene Structure to Phenotypic Expression, Limone S.G., 1988). The modified apolipoprotein particles may thus remain in the circulation for very long periods, thus exerting their arterial protective activity in a better way, compared to normal AI particles.

The therapeutic use of apolipoprotein AI or of its AI-M mutant is presently limited by the lack of a method allowing the preparation of said apolipoproteins in sufficient amount and in a suitable form.

The difficulty of producing apolipoprotein AI and particularly AI Milano (AI-M) from plasma fractionation is quite considerable (Franceschini et al., J.Biol.Chem., 260: 16321-16325, 1985). Furthermore there are a series of risks associated with plasma fractionation products, such as contamination with infectious agents and availability of starting material, that are essential to avoid.

Several attempts have been made to produce human Apo AI, by way of the recombinant DNA technology. In WO 88/03166 the preparation of Apo AI from E.coli is described. The process describes a chimeric polypeptide where the Apo AI moiety is fused to the N-terminal amino acid residues of beta-galactosidase or to one or more IgG-binding domains of Protein A, or to the pro sequence of human Apo AI.

The above methods suffer from the fact that E.coli is used as the transformant organism. The expressed products are not the mature human Apo AI but contain substantial sequences of heterologous origin. Furthermore the products are not secreted from cells but accumulate intracellularly in the E.coli host organism, thus enhancing the risk of enzymatic degradation of the expressed product.

A more convenient system for expression of mammalian polypeptides seems to be eukaryotic cells and several attempts have been made to express foreign genes in eukaryotes, especially in yeast. Expression of interferon in yeast is described in European patent publication No. 0060057 and expression and secretion in yeast of proteins heterologous to yeast is described in European patent publication Nos. 0088632, 0116201 and 0123544. WO 87/02062 discloses the expression of an apolipoprotein in yeast cells

The object of the present invention is to provide Apo AI and Apo AI-M which are generated in high yields in yeast with correctly folded structure and the properties of natural human Apo AI, namely structural features, lipid binding and activation of fibrinolysis and of the lecithin: cholesterol acyl transferase (LCAT) (Mahley et al., J.Lipid. Res., 25: 1277-1294, 1984).

According to the present invention the genes encoding the Apo AI and Apo AI-M, were provided with DNA-sequences encoding yeast-recognizable secretion and processing signals fused upstream to the gene for the mature proteins. In particular a modified MF α 1 leader sequence was used in which the last residues where: HisGlySerLeuAspLysArg.

In this modified MF α 1 leader the processing signal was the dibasic sequence Lys-Arg, which is then fused to the 5' terminus of the Apo AI gene. It was found that the leader sequence was cleaved off in all the constructions, i.e. cleavage occured at the dibasic sequence Lys-Arg upstream to all the Apo AI and Apo AI-M polypeptides.

This invention provides a method for producing Apo AI or Apo AI-M in yeast. By this method a yeast strain transformed with an expression vector comprising a DNA-sequence encoding the Apo AI or Apo AI-M proteins is cultivated in a suitable culture medium and the Apo AI or Apo AI-M molecules are recovered from the culture medium.

When cultivating such transformed yeast strains, high yields of the Apo AI or Apo AI-M are isolated from the culture broth.

The expression products were isolated and Apo AI immunoreactive material was purified and characterized by microsequence analysis. It was found that this polypeptide has the same characteristics as the natural Apo AI.

The expression vectors of the invention comprise a replication system for stable maintenance in a yeast host, DNA-sequences encoding the Apo AI or Apo AI-M and promoter and terminator sequences.

The expression vector may, upstream to DNA-sequence encoding the desired product, contain a preregion ensuring direction of the expressed product into the yeast secretory pathway and secretion of the expressed product into the growth medium. This preregion, which might be a naturally occurring signal or leader peptide or a synthetic sequence providing secretion, is generally cleaved from the desired product during secretion, leaving the mature product ready for isolation from the culture broth.

A well suited leader sequence for yeast is the yeast MF $\alpha$ 1 leader sequence (Kurjan, j. and Herskowits, I., Cell 30: 933-946, 1982).

The expression vector may be a plasmid capable of replication in the host microorganism or capable of integration into the host chromosome. The vector employed may code for expression of repeated copies of the desired DNA-sequence, each separated by selective cleavage sites.

The expression of the desired DNA-sequence will be under control of a promoter sequence, correctly positioned to the DNA-sequence encoding the desired product to result in expression of the desired product in the host organism. Preferably a promoter from a gene indigenous to the yeast host is used, e.g. the promoter of TPI (triose phosphate isomerase) gene or the MF $\alpha$ 1-promoter.

The DNA-sequence for the desired product will be followed by a transcription terminator sequence, preferably a terminator sequence from a gene indigenous to the yeast host, e.g. the terminator of the TPI-gene or the MF $\alpha$ 1-gene.

The present invention describes also the manipulation of the previously cloned Apo AI and Apo AI-M sequences (Sharpe CR, Sidoli A, Shelley CS, Lucero MA, Shoulders CC and Baralle FE, Nucl. Acids Res., 12: 3917-3932, (1984) to make them suitable for secretion in yeast, using a chemically synthesized adaptor of 46 nucleotides, cloned in the unique NcoI site of Apo AI.

The Apo AI-M mutation was generated by partially cloning the mutant Apo AI-M gene from a carrier and by replacing part of it into the wild type sequence.

In the drawings:
- Figure 1 shows the sequence of the 46-nucleotide adapter containing the end of the MF $\alpha$ 1 leader sequence and the amino-terminal sequence of mature Apo AI.
- Figure 2 shows the construction of the plasmid pUC/AF-AI from pUC8.
- Figure 3 shows the construction of pUC/AF-AI-M starting from the plasmid of Figure 2.
- Figure 4 shows the preparation of the expression vectors pYES/AI and pYES/AI-M, starting from pYES and from the plasmids of figures 2 and 3.

## Construction of the Apo AI and Apo AI-M sequences

The Apo AI sequences were obtained from our previously described cDNA clone (Sharpe et al., Nucl.Acids Res., 1984), from which the sequence coding for the signal peptide and the propeptide were removed and modified with the following strategy.

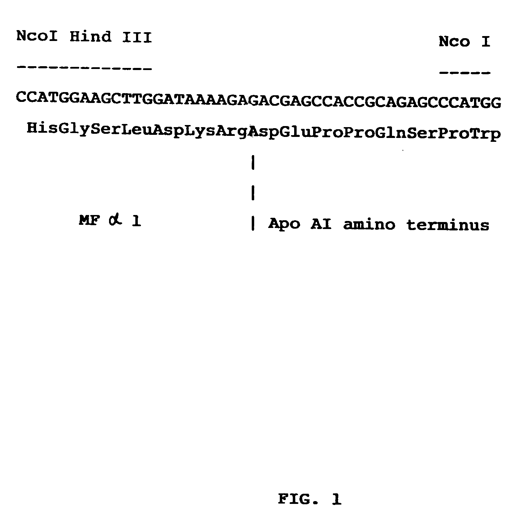

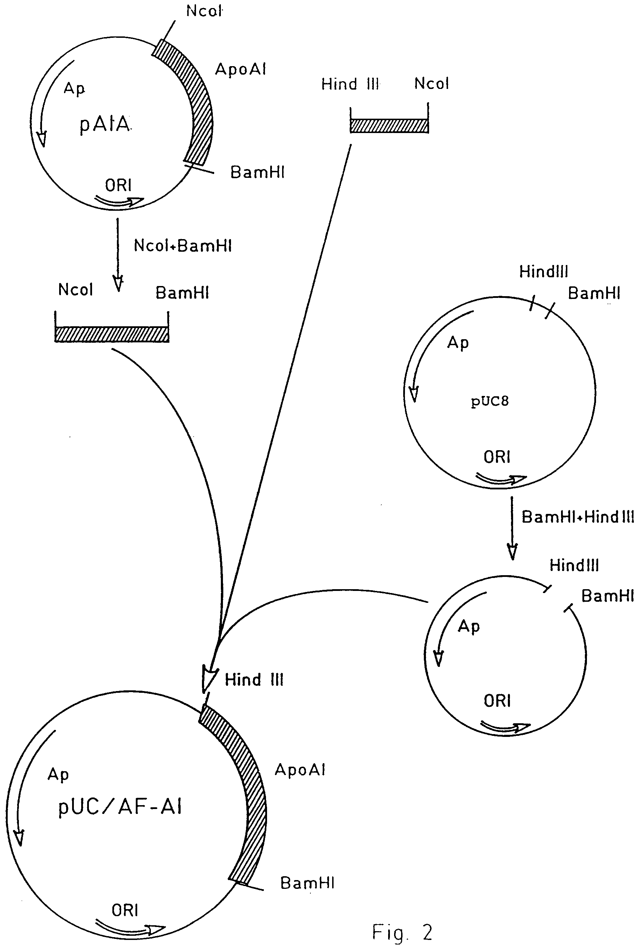

The Nco I - BamHI fragment from the original cDNA sequences (plasmid pAIA) was ligated simultaneously with a chemically synthesized fragment Hind III - NcoI, containing the end of the MF $\alpha$ 1 leader sequence and the amino terminal sequence of mature Apo AI (fig. 1) and a Hind III - Bam HI digested pUC 8 (see fig. 2). This clone is named pUC/AF-AI.

The Apo AI-M sequence was obtained by cloning part of the gene from an Apo AI-M carrier. The methodology used was as follows:

a) DNA was prepared from peripheral white blood cells following standard methods (Kunkel LM, Smith KD, Boyer SH, Borgaonkar DS, Watchel SS, Miller BJ, Berg WR, Jones HW and Rary JM., Proc. Natl. Acad. Sci. USA, 1245-1249, 1977).

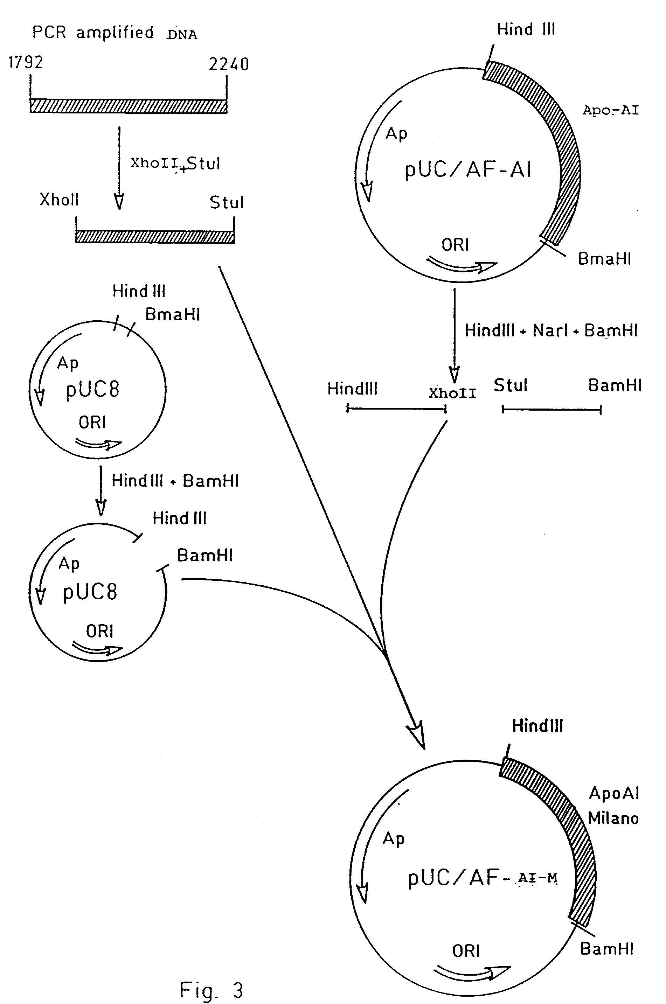

b) The fragment between nucleotides 1792 and 2240 (see fig. 3) (Shoulders CC, Kronblihtt AR, Munro BS and F.E. Baralle Nucl. Acids Res., 11: 2827-2837, 1983) was produced by the amplification procedure known as Polymerase Chain Reaction (PCR. Saiki RK, Gelfand DH, Stoffel S, Scharf S, Higuchi R and Horn GT. Science 239: 487-491, 1988), using two synthetic oligonucleotides as primers in the enzymatic reaction on the Apo AI-M genomic DNA, prepared from a carrier, having the following sequences:

5'-CTGAGGCAAGAGATGAGCAA-3'; 5' end in 1792.
5'-CTCAGGAAGCTGACCTTGAA-3'; 5' end in 2240.

c) This fragment was subjected to Xho II and Stu I digestions and the resulting product was ligated with the Hind III - Xho II and Stu I - BamHI fragments from pUC/AF-AI into a pUC-8 BamHI-HindIII linearized vector.

The obtained clone is named pUC/AI-M.

## Plasmid construction

Genes encoding the human Apo AI and Apo AI-M were combined with fragments coding for the TPI promoter (TPI$_p$) (T. Alber and G. Kawasaki. J.Mol. Applied Genet. I: 419-434, 1982), the MF α 1 leader sequence (J. Kurkian and I. Herskowitz., Cell 30: 933-943, 1982) and the transcription termination sequence from TPI of S. cerevisiae TPI$_t$.

These fragments provide sequences to ensure a high rate of transcription for the apo AI encoding gene and also provide a presequence which can effect the localization of Apo AI and Apo AI-M polypeptides into the secretory pathway and its eventual excretion into the growth medium.

The expression plasmids further comprise the yeast 2μ origin of replication and a selectable marker LEU2.

During in vivo maturation of α-factor in yeast, the last (C-terminal) six amino acids of the MF α 1 leader peptide (Lys-Arg-Glu-Ala-Glu-Ala) are removed from the -factor precursor by the sequential action of an endopeptidase recognizing the Lys-Arg sequence and an aminodipeptidase which removes the Glu-Ala residues (Julius D, Blair L, Brake A, Sprangue G and Thorner J, Cell 32: 839-852, 1983). To eliminate the need for the yeast aminodipeptidase, the sequence coding for the C-terminal Glu-Ala-Glu-Ala of the MF α 1 leader was removed via in vitro mutagenesis.

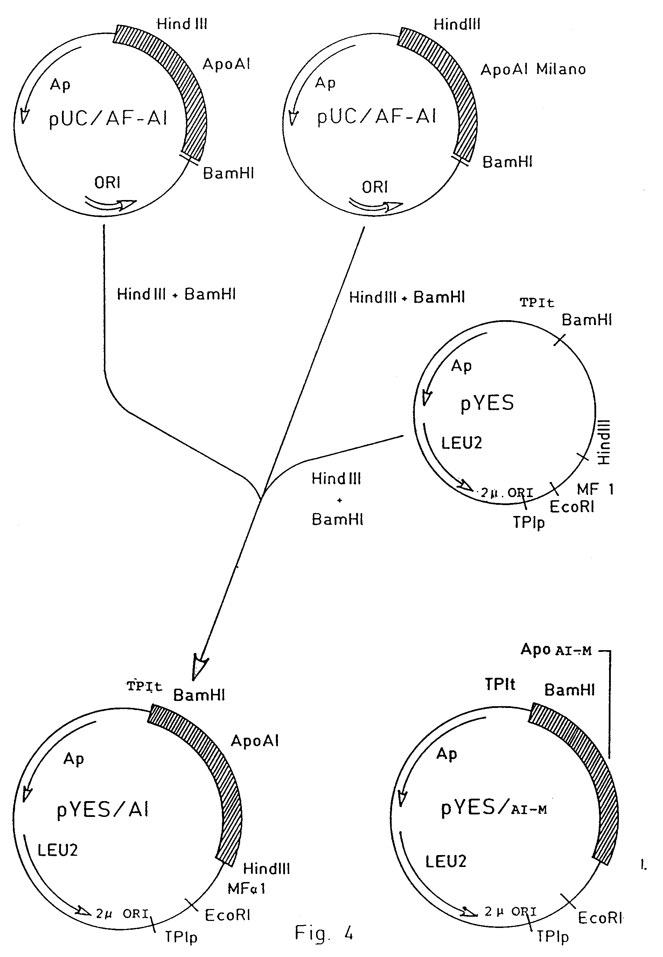

The Hind III-Bam HI fragments from pUC/AF-AI and pUC/AF-AI-M were cloned in the Hind III-Bam HI linearized pYES vectors, as shown in fig. 4. The resulting plasmids for yeast transformation and expression of Apo AI and Apo AI-M genes, are called pYES/AI and pYES/AI-M, respectively.

## Transformation

Plasmids prepared as above described were transformed into S. cerevisiae strains carrying deletions into the TPI gene by selecting for growth on glucose. Such strains are normally unable to grow on glucose as the sole carbon source and grow very slowly on galactose lactate medium. This defect is due to a mutation in the triose phosphate isomerase gene, obtained by deletion and replacement of a major part of this gene with the S. cerevisiae LEU 2 gene. Because of the growth deficiency there is a strong selection for a plasmid containg a gene coding for TPI.

## Expression of the Apo AI and Apo AI-M precursors in yeast

The yeast strains containing plasmids encoding for the apolipoproteins were grown on YPD medium (Sherman, F. et al., Methods in Yeast Genetics, Cold Spring Harbor Laboratory 1981). For each strain, two cultures of 1 litre each in a baffled flask of 2 litres were shaken at 30°C until they reached an OD at 600 nm of approx. 15 (approx. 48 h). After centrifugation the supernatant was removed for further analysis.

## Purification of Apo AI and Apo AI-M culture broth

The culture broth was centrifuged at 15,000 rpm for 30 min. and the supernatant dialysed against 0.15 M NaCl, 0.05 M K-phosphate buffer, 0.05% EDTA, pH 7.4 (buffer A).

Triglyceride-rich particles (TGRP) were isolated from commercial phospholipid-triglyceride emulsions, by ultracentrifugal washing at 4°C, and resuspended in buffer A. The supernatant from culture broth was incubated with TGRP at 37°C for 60 min., with gentle agitation and incubations terminated by immersion in an ice-water bath. TGRP were reisolated by ultracentrifugation at 27,000 rpm for 35 min. at 40°C and delipidated with diethylether:ethanol 3:1. The precipitated protein was collected by low speed centrifugation and passed through a Sephacryl S-200 column and/or an anti-apo AI-Sepharose column.

## Structural characterization of the recombinant apolipoprotein

The structural features of the recombinant Apo AI and AI-M were tested by aminoacid analysis and protein microsequencing, confirming the purity of the apolipoprotein preparations. The secondary structure was analyzed by circular dichroism, indicating that the recombinant apolipoprotein are properly folded: the α'-helix content was calculated as 52% and 43% for recombinant Apo AI and Apo AI-M, respectively, as a 49% content for extractive Apo AI. Thus recombinant AI-M, as extractive AI-M (Franceschini et al., J. Biol. Chem., 260: 16321-16325, 1985), has a lower α-helix content compared to normal AI.

Biological evaluation of recombinant Apo AI-M vs Apo AI

All reported experiments were carried out by comparing Apo AI and Apo AI-M, obtained from S. cerevisiae, with human Apo AI, extracted from plasma. Human Apo AI was prepared from human HDL, separated by preparative ultracentrifugation. Following delipidazation of the isolated HDL fraction, Apo AI was separated by gel filtration and ion exchange chromatography (Baker et al., Biochemistry, 12: 3866-3871, 1983). In vitro studies

Activation of fibrinolysis

Agents promoting the conversion of plasminogen to plasmin, thus initiating fibrinolysis, are gaining increased interest in the treatment of myocardial infarction (Laffell and Braunwald, N. Engl. J. Med., 311: 710-717, 1984). Previous data have shown that the Apo AI can activate fibrinolysis in vitro (Saku et al., Thromb. Res., 39: 1-8, 1985).

Experiments were carried out by the fibrin plate method (Glas-Greenwalt et al., J. Lab. Clin. Med., 104: 962-976, 1984) in order to evaluate the potentiation or inhibition of urokinase activity.

A bovine (plasminogen-rich) fibrinogen solution, at a final concentration of 0.1% in a volume of 6 ml, was clotted with 0.2 ml of a bovine thrombin solution (20 NIH Units/ml). Recombinant Apo AI and Apo AI-M (concentrations between 10 and 500 $\mu$/g), diluted in Tris buffer were rapidly mixed with urokinase (f.c. 3.3 CAT U/ml) and applied to the fibrin plates. These were incubated for 17 h a 37°C. The lysis area measurements indicate that both proteins significantly increased the urokinase lysis areas, at the highest tested doses, by 64.0% and 67.9% respectively in the case of Apo AI (vs 59.7% and 66.2% for extractive Apo AI; Saku et al., Thromb. Res., 39: 1-8, 1985) and by 96.7% and 112.1% in the case of Apo AI-M (Table I). In this system, therefore, Apo AI-M appears to be significantly more potent than Apo AI.

Removal of cholesterol from cultured macrophages

The so called "reverse cholesterol transport" (RCT), i.e. the transport of cholesterol from tissues to plasma, is believed to be regulated by HDL, acting as the acceptor lipoprotein (Glomset J., Adv. Int. Med., 25: 91-116, 1980). Since Apo AI is believed to be responsible for the removal capacity of HDL, experiments were carried out with J-774 mouse macrophages equilibrated with $^3$H cholesterol. The monolayers were incubated with "Minimum Essential Medium", solvent-extracted delipidized calf serum protein, egg phosphatidylcholine (PC) and esterified cholesterol (Rothblat et al., In vitro, 12: 554-557, 1976). Equilibration with $^3$H cholesterol was for 48 h, after which the radioactive medium was removed, the cells extensively washed and added with fresh medium, supplemented with 5 mg/ml delipidized calf serum protein. After overnight incubation, the cells were again extensively washed and incubated with HEPES-buffered Williams Medium E in 35 mm flat dishes, with the addition of 2 ml of each acceptor particle. Each 90 min., 0.1 ml aliquots of the incubation medium were taken, in the fist 6 h and then every 3 h, for a total 24 h period and counted in order to determine the amount of labelled cholesterol released by the cells (Rothblat and Phillips, J. Biol. Chem. , 250: 4775-4782, 1982).

Proteoliposomes containing recombinant Apo AI or Apo AI-M were prepared with egg PC according to the cholate dialysis procedure, as described by Mats and Jonas (J. Biol. Chem., 257: 4535-4540, 1982). Mixtures were generally of apolipoprotein/phospholipid/cholate (mass ratio of 1/2.5/1.5-2.5) in 10mM Tris/1mM EDTA/1mM NaN$_3$/150 mM NaCl (pH 8.0). In order to size the apolipoprotein/phospholipid particles, a gel filtration on Sepharose CL/4B (1.5 x 80 cm column) eluted with 0.15 M NaCl/0.02% EDTA, pH 7 was carried out. Peak fractions were concentrated on ultrafiltration cells (Amicon model 52). By determination of the chemical composition and molecular microscopic features of the particles containing Apo AI or Apo AI-M, no significant differences could be found. To evaluate the capacity to remove cell cholesterol, two different concentrations of the apolipoprotein component (50 and 100 $\mu$g/ml, i.e. corresponding to concentrations of PC/ml medium of 150 and 300 $\mu$g) were added to the cells. Since, under these experimental conditions, the unidirectional flux of cholesterol is first-order with respect to cell free cholesterol concentrations, it is possible to calculate a half-time for the cholesterol efflux (t1/2) in the different conditions.

A shown in Table 2, at both concentrations of apolipoprotein in the liposomes, the efflux of free cholesterol from J-774 cells was considerably faster with Apo AI-M, compared to Apo AI. Again, no difference could be detected between the recombinant and extractive Apo AI (t1/2: 17.9±3.2).

In vivo study of arterial atherosclerosis regression

This experiment evaluated the capacity of extractive Apo AI and recombinant Apo AI-M continuous infusions for 4 weeks, to reduce the arterial lesions, established with a 0.5% cholesterol rich diet for 2 months in rabbits. Twenty-five New Zealand male rabbits (starting weight 2.2-2.5 kg) received a 0.5% cholesterol rich diet for 2 months. At this time 4 animals were sacrified. Their aortas showed 45.3±7.6% involvement with atherosclerotic lesions. At this point, the animals were divided into three groups, each of 7 animals. Group 1 was placed on a standard, cholesterol-free diet with no treatment. Group 2 was continuously infused with Apo AI, by way of a subcutaneously implanted Alzet[R] Minipump, connected to an ear vein. Group 3 was treated in the same way with Apo AI-M. The daily total protein dose was 7 mg/rabbit for each of the two apolipoproteins.

After 4 weeks of treatment, both the extent of aortic fatty lesions and the aortic lipid content were evaluated in the three groups. The data (Table 3) show a more than 30% regression of lesions with Apo AI, and a more than 60% regression with Apo AI-M, with a corresponding highly significant reduction of aortic total cholesterol and cholesteryl esters in both groups, Apo AI-M being significantly more effective, compared to normal Apo AI.

The reported findings confirm the remarkable activity of Apo AI, in an in vivo condition (Badimon et al., Arteriosclerosis, 7: 522a, 1987) and indicate that Apo AI-M, probably because of its physico-chemical properties, is more effective in inducing the regression of atherosclerotic lesions, compared to normal human AI.

**Table 1.** Effect of the addition of Apo AI or of Apo AI-M on urokinase activated fibrinolysis (X±SD; n = 4 per dose applied)

---

<u>Total protein applied</u> (µg)

| | 0 | 10 | 20 | 50 | 100 | 250 | 500 |
|---|---|---|---|---|---|---|---|
| | | | | Lysis area ($mm^2$) | | | |
| **Apo AI** | 562±31 | 582±34 | 601±29 | 666±22* | 702±39* | 922±55*** | 944±76*** |
| **Apo AI-M** | 578±26 | 596±17 | 636±33 | 751±47** | 804±44**° | 1137±66**°° | 1226±51***°°° |

---

*p < 0.05; **p < 0.01; ***p < 0.001 vs control

°p < 0.05; °°p < 0.01; °°°p < 0.001 vs Apo AI

EP 0 469 017 B1

**Table 2.** Comparison of half-times for cholesterol efflux from J-774 cells (X±SD)

------------------------------------------------------------------------------

|  | Apo AI: egg PC acceptor | Apo AI-M: egg PC acceptor |
|---|---|---|
| Half-time (t 1/2 h) | 18.3±4.2 (n=11) | 12.1±2.2** ( n=12) |

------------------------------------------------------------------------------

*p < 0.01

EP 0 469 017 B1

Table 3. Extent of aortic lesions, cholesterol and cholesteryl ester aortic content in rabbits after Apo AI and Apo AI-M infusion (n=7 per group, X±SD)

| | % aortic lesions | Cholesterol | Cholesteryl esters |
|---|---|---|---|
| Group 1 (control) | 44±4 | 16.4±2.1 | 14.8±1.6 |
| Group 2 (Apo AI) | 28±3** | 7.9±1.4 | 6.2±0.8 |
| Group 3 (Apo AI-M) | 17±2**°° | 5.3±0.7**°° | 4.0±0.4**°° |

**p < 0.01 vs group 1; °°p<0.01 vs group 2

## Claims

1. An expression vector capable of expressing, in a transformed yeast, apolipoprotein AI or apolipoprotein AI-M (Milano) comprising a promoter sequence autologous to the yeast to be transformed, a DNA sequence coding for Apo AI or Apo AI-M, a sequence coding for a leader which is the modified MF $\alpha$ 1

leader sequence encoding for the residue His Gly Ser Leu Asp Lys Arg, and a transcription terminator sequence autologous to the yeast to be transformed.

2. An expression vector according to claim 1 capable of expressing Apo AI or Apo AI-M, which is derived form plasmid pUC8.

3. An expression vector according to any one of the previous claims containing a Hind III NcoI fragment of the sequence

**NcoI Hind III                                        Nco I**

**--------------                                        -----**

**CCATGGAAGCTTGGATAAAAGAGACGAGCCACCGCAGAGCCCATGG**

followed by the NcoI-Bam HI of the Apo AI cDNA, or the XhOII-StuI of the ApoAI-M cDNA, the transcription termination sequence from TPI of S.cerevisiae TPI$_t$, the Hind III-Bam HI fragment of pUC 8 containing the ampicillin resistance gene, the LEU2 marker from the 2$\mu$ plasmid of S.cerevisiae, the TPI promoter and the MF$\alpha$I leader sequence, in that order.

4. A synthetic DNA encoding for the residue His Gly Ser Leu Asp Lys Arg Asp Glu Pro Pro Gln Ser Pro Trp having the following sequence
    CCATGGAAGCTTGGATAAAAGAGACGAGCCACCGCAGAGCCCATGG

5. A host yeast transformed with the expression vector of claims 1-4.

6. A host yeast according to claim 5 which is a strain of <u>Saccharomyces cerevisiae</u>.

7. Process for the preparation of recombinant apolipoprotein AI or apolipoprotein AI-M comprising the cultivation of a host yeast according to claim 5 and the isolation of the desired apolipoprotein from the culture broth.

**Patentansprüche**

1. Expressionsvektor, der in der Lage ist, in transformierter Hefe Apolipoprotein AI oder Apolipoprotein AI-M (Milano) zu exprimieren, umfassend eine bezüglich der Hefe, die transformiert werden soll, autologe Promotorsequenz, eine DNA-Sequenz, die für Apo AI oder Apo AI-M kodiert, eine Sequenz, die für eine Leadersequenz kodiert, bei der es sich um die modifizierte MF $\alpha$ 1 Leadersequenz handelt, die für die Aminosäurereste His Gly Ser Leu Asp Lys Arg kodiert und eine bezüglich der Hefe, die transformiert werden soll, autologe Transkriptionsterminator-Sequenz.

2. Expressionsvektor nach Anspruch 1, der in der Lage ist, Apo AI oder Apo AI-M zu exprimieren und der von dem Plasmid pUC8 abgeleitet ist.

3. Expressionsvektor nach einem der vorstehenden Ansprüche, der ein Hind III/Nco I-Fragment der Sequenz

**NcoI Hind III                                        Nco I**

**--------------                                        -----**

**CCATGGAAGCTTGGATAAAAGAGACGAGCCACCGCAGAGCCCATGG**

enthält, gefolgt in dieser Reihenfolge vom Nco I/Bam HI-Fragment der Apo AI cDNA oder vom Xho II/Stu I-Fragment der Apo AI-M cDNA, der TPI-Transkriptionsterminations-Sequenz von S.cerevisiae

TPI$_t$, dem Hind III/Bam HI-Fragment von pUC8, das das Ampicillin-Resistenzgen enthält, den LEU2-Marker des 2μ-Plasmids von S. cerevisiae, den TPI-Promotor und die MFαI-Leadersequenz.

4. Synthetische DNA, die für die Aminosäurereste His Gly Ser Leu Asp Lys Arg Asp Glu Pro Pro Gln Ser Pro Trp kodiert und die folgende Sequenz hat:
CCATGGAAGCTTGGATAAAAGAGACGAGCCACCGCAGAGCCCATGG

5. Hefewirtszelle, die mit dem Expressionsvektor nach den Ansprüchen 1 bis 4 transformiert wurden.

6. Hefewirtszelle nach Anspruch 5, bei der es sich um einen Stamm von Saccharomyces cerevisiae handelt.

7. Verfahren zur Herstellung von rekombinantem Apolipoprotein AI oder Apolipoprotein AI-M, umfassend die Kultivierung einer Hefewirtszelle nach Anspruch 5 und die Isolierung des gewünschten Apolipoproteins aus der Kulturflüssigkeit.

**Revendications**

1. Un vecteur d'expression capable d'exprimer, dans une levure transformée, de l'apolipoprotéine AI ou de l'apolipoprotéine AI-M (Milano) comprenant une séquence promotrice autologue à la levure à transformer, une séquence d'ADN codant pour l'Apo AI ou l'Apo AI-M, une séquence codant pour un leader qui est la séquence leader MF α 1 modifiée codant pour le résidu His Gly Ser Leu Asp Lys Arg, et une séquence terminatrice de transcription autologue à la levure à transformer.

2. Un vecteur d'expression selon la revendication 1, capable d'exprimer l'Apo AI ou l'Apo AI-M, qui est dérivé du plasmide pUC8.

3. Un vecteur d'expression selon l'une quelconque des revendications précédentes contenant un fragment Hind III NcoI de la séquence

```
NcoI Hind III                          Nco I
_____                        _____
```

CCATGGAAGCTTGGATAAAAGAGACGAGCCACCGCAGAGCCCATGG

suivi par le NcoI-Bam HI de l'ADNc de Apo AI, ou le XhOII-StuI de l'ADNc de Apo AI-M, la séquence terminatrice de transcription provenant du TPI de TPIt de S.cerevisiae, le fragment Hind III-Bam HI de pUC 8 contenant le gène de résistance à l'ampicilline, le marqueur LEU2 provenant du plasmide 2μ de S.cerevisiae, le promoteur TPI et la séquence leader MFαI, dans cet ordre.

4. Un ADN synthétique codant pour le résidu His Gly Ser Leu Asp Lys Arg Asp Glu Pro Pro Gln Ser Pro Trp ayant la séquence suivante :
CCATGGAAGCTTGGATAAAAGAGACGAGCCACCGCAGAGCCCATGG

5. Une levure hôte transformée par le vecteur d'expression des revendications 1-4.

6. Une levure hôte selon la revendication 5 qui est une souche de Saccharomyces cerevisiae.

7. Procédé de préparation d'apolipoprotéine AI ou d'apolipoprotéine AI-M recombinante comprenant la culture d'une levure hôte selon la revendication 5 et l'isolement de l'apolipoprotéine désirée à partir du bouillon de culture.

NcoI Hind III                                    Nco I

‒ ‒ ‒ ‒ ‒ ‒ ‒ ‒ ‒ ‒ ‒                            ‒ ‒ ‒ ‒ ‒

CCATGGAAGCTTGGATAAAAGAGACGAGCCACCGCAGAGCCCATGG

HisGlySerLeuAspLysArgAspGluProProGlnSerProTrp

|

|

MF α 1          | Apo AI amino terminus

FIG. 1

Fig. 2

Fig. 3

Fig. 4